# EUROPÄISCHE PATENTANMELDUNG

(11) **EP 2 316 502 A1**
(43) Veröffentlichungstag der Anmeldung: **04.05.2011**
(21) Anmeldenummer: 10189607.4
(22) Anmeldetag: 02.11.2010
(51) Int. Cl.: A61M 1/10

(54) **Dialysevorrichtungen**

(30) Priorität: 03.11.2009 DE 102009051805
(71) Anmelder: Gambro Lundia AB, 220 10 Lund (SE)
(72) Erfinder: Wagner, Steffen, 72469, Meßstetten (DE); Flieg, Ralf, 72414, Rangendingen (DE)
(74) Vertreter: Perchenek, Nils

(57) **Zusammenfassung**

Die vorliegende Erfindung bezieht sich auf Dialysevorrichtungen, die peristaltische Schlauchpumpen (1,2,3,6,8,10,11,18,21) linearer Bauart mit piezoelektrischen Aktuatoren aufweisen.

## Beschreibung

### Technisches Gebiet der Erfindung

Die vorliegende Erfindung bezieht sich auf Dialysevorrichtungen, die peristaltische Schlauchpumpen linearer Bauart mit piezoelektrischen Aktuatoren aufweisen.

### Hintergrund der Erfindung

In heutigen Dialysesystemen erfolgt die Flüssigkeitsförderung in der Regel durch radiale Schlauchquetschpumpen, die eingesetzt werden, um Blut, Dialyseflüssigkeit bzw. zu deren Herstellung eingesetzte Flüssigkeiten bzw. Konzentrate und Filtrat zu pumpen.

Nachteilig an den bislang eingesetzten radialen Schlauchquetschpumpen sind deren Baugröße und Gewicht, die zum beträchtlichen Raumbedarf und Gewicht gängiger Dialysesysteme beiträgt, welche dazu führt, dass diese Systeme häufig sehr sperrig und schwer sind nur mühsam transportiert werden können. Zudem treten bei diesem Pumpentyp im Betrieb unangenehme Lärmentwicklung und hohe Stromaufnahme und damit verbundene Wärmeentwicklung auf.

Es wäre daher wünschenswert, insbesondere auch im Hinblick auf die Entwicklung von Systemen für die Heimdialyse bzw. die Entwicklung tragbarer Dialysesysteme bzw. tragbarer künstlicher Nieren, Vorrichtungen mit alternativen Pumpsystemen auszurüsten, die diese Nachteile nicht aufweisen.

### Zusammenfassung der Erfindung

Es wurde nun gefunden, dass peristaltische Schlauchpumpen linearer Bauart mit piezoelektrischen Aktuatoren vorteilhaft die üblichen radialen Schlauchquetschpumpen in Dialysesystemen ersetzen können.

Die vorliegende Erfindung stellt Dialysesysteme zur Verfügung, die Schlauchpumpen linearer Bauart mit piezoelektrischen Aktuatoren aufweisen. Die erfindungsgemäßen Dialysesysteme zeichnen sich gegenüber den Systemen des Standes der Technik durch geringere Baugröße und geringeres Gewicht aus, entwickeln praktisch keinen Lärm und haben eine geringe Stromaufnahme und dadurch auch geringe Wärmeentwicklung.

Gegenstand der Erfindung ist auch die Verwendung von Schlauchpumpen linearer Bauart mit piezoelektrischen Aktuatoren in Dialysesystemen.

### Kurze Beschreibung der Zeichnungen

Fig. 1 zeigt schematisch eine Ausführungsform eines erfindungsgemäßen Dialysesystems, die für einen Einsatz in der Intensivmedizin geeignet ist;
Fig. 2a und 2b zeigen schematisch andere Ausführungsformen eines erfindungsgemäßen Dialysesystems, die für einen Einsatz in der chronischen Dialyse oder der Heimdialyse geeignet sind;
Fig. 3 zeigt schematisch eine andere Ausführungsform eines erfindungsgemäßen Dialysesystems, die für einen Einsatz in der Heimdialyse oder für einen Einsatz als tragbares Dialysesystem geeignet ist;
Fig. 4 zeigt schematisch ein Zusatzmodul für ein Dialysesystem, das für die Steuerung der Blutgerinnung während der Dialyse eingesetzt werden kann;
Fig. 5a und 5b zeigen schematisch andere Ausführungsformen eines erfindungsgemäßen Dialysesystems, die für einen Einsatz in der Peritonealdialyse geeignet sind.

### Ausführliche Beschreibung

Bevorzugte Ausführungsformen der vorliegenden Erfindung werden jetzt ausführlicher unter Bezugnahme auf die beigefügten Zeichnungen beschrieben werden.

Für den Einsatz in den erfindungsgemäßen Dialysesystemen geeignete peristaltische Schlauchpumpen linearer Bauart mit piezoelektrischen Aktuatoren (im Folgenden kurz als "Piezopumpen" bezeichnet) sind dem Fachmann im Prinzip bekannt. So können beispielsweise Pumpen verwendet werden, wie sie in GB-A 2 238 833 oder WO 97/42412 offenbart sind. Ebenfalls denkbar, aber weniger bevorzugt, ist auch ein Einsatz von Pumpen der Art, wie sie aus WO 2004/071684 A1, US 4 432 699 A oder DE 10 2007 019 433 A1 hervorgehen.

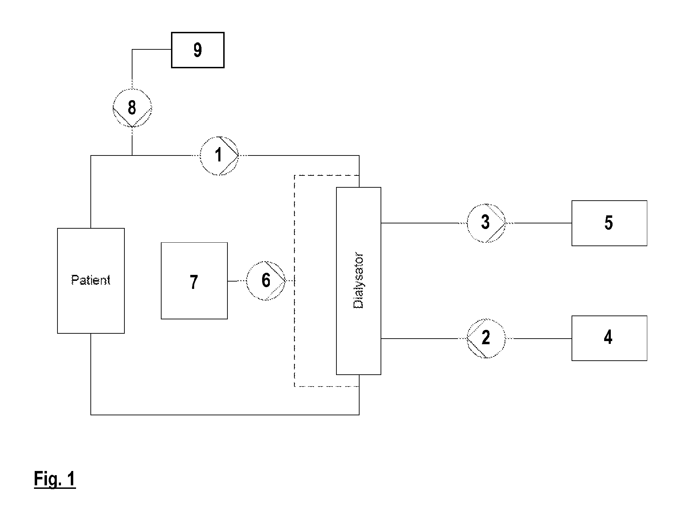

Fig.1 zeigt schematisch eine Ausführungsform eines erfindungsgemäßen Dialysesystems, die für einen Einsatz in der Intensivmedizin geeignet ist. Eine Piezopumpe (**1**) ist dafür vorgesehen, das Blut des Patienten durch den extrakorporalen Kreislauf zu pumpen. Piezopumpen (**2,3**) sind dafür vorgesehen, Dialysat aus einer Quelle für Dialysat (**4**), z.B. einem Beutel oder einer Vorrichtung zur on-line Herstellung von Dialysat, durch den Dialysator und in einen Behälter (**5**) für verbrauchtes Dialysat zu pumpen. Eine weitere Piezopumpe (**6**) ist dafür eingerichtet, bei Bedarf Substitutionsflüssigkeit aus einer Quelle (**7**), z.B. einem Vorratsbehälter oder einer Vorrichtung zur on-line Herstellung von Substitutionsflüssigkeit, dem extrakorporalen Blutkreislauf zuzuführen, z.B. wenn das Dialysesystem im Hämodiafiltrationsmodus (HDF) oder im Hämofiltrationsmodus (HF) betrieben wird, wahlweise vor dem Dialysator ("pre-dilution mode") oder hinter dem Dialysator ("post-dilution mode"). Eine weitere Piezopumpe (**8**) ist dafür vorgesehen, Infusionslösung (beispielsweise Medikamentenlösungen) aus einer Quelle (**9**), z.B. einem Vorratsbehälter, dem extrakorporalen Blutkreislauf zuzuführen.

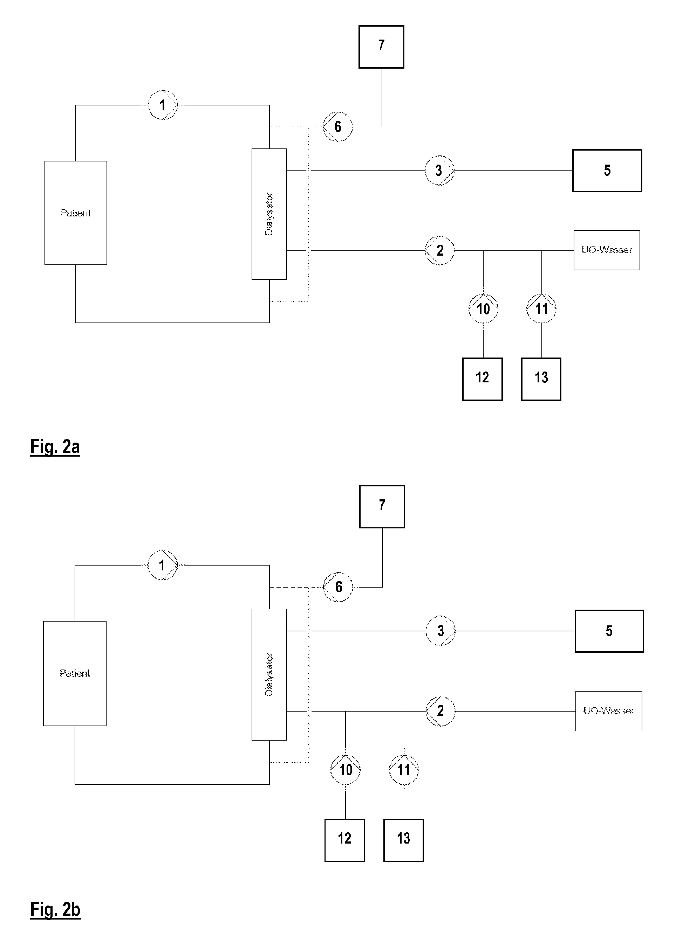

Fig. 2a und 2b zeigen schematisch Ausführungsformen eines erfindungsgemäßen Dialysesystems, die für einen Einsatz in der chronischen Dialyse oder der Heimdialyse geeignet sind. Eine Piezopumpe (**1**) ist dafür vorgesehen, das Blut des Patienten durch den extrakorporalen Kreislauf zu pumpen. Piezopumpen (**2,3**) sind dafür vorgesehen, Dialysat durch den Dialysator und in einen Behälter (**5**) für verbrauchtes Dialysat zu pumpen. Das Dialysat wird in dieser Ausführungsform erzeugt aus durch Umkehrosmose demineralisiertem Wasser (UO Wasser) und Dialysekonzentraten (**12,13**), die über Piezopumpen (**10,11**) beigemischt werden. Die Zumischung der Konzentrate kann vor der ersten Dialysatpumpe (**2**) erfolgen, wie in Fig. 2a dargestellt, oder dahinter, wie in Fig. 2b gezeigt. Eine weitere Piezopumpe (**6**) ist dafür eingerichtet, bei Bedarf Substitutionsflüssigkeit aus einer Quelle (**7**) dem extrakorporalen Blutkreislauf zuzuführen, z.B. wenn das Dialysesystem im Hämodiafiltrationsmodus (HDF) oder im Hämofiltrationsmodus (HF) betrieben wird, wahlweise vor dem Dialysator ("pre-dilution mode") oder hinter dem Dialysator ("post-dilution mode"). Alternativ kann Piezopumpe (**6**) auch dafür verwendet werden, anstelle von Substitutionsflüssigkeit eine Infusionslösung (beispielsweise Medikamentenlösungen) aus der Quelle (**7**) dem extrakorporalen Blutkreislauf zuzuführen.

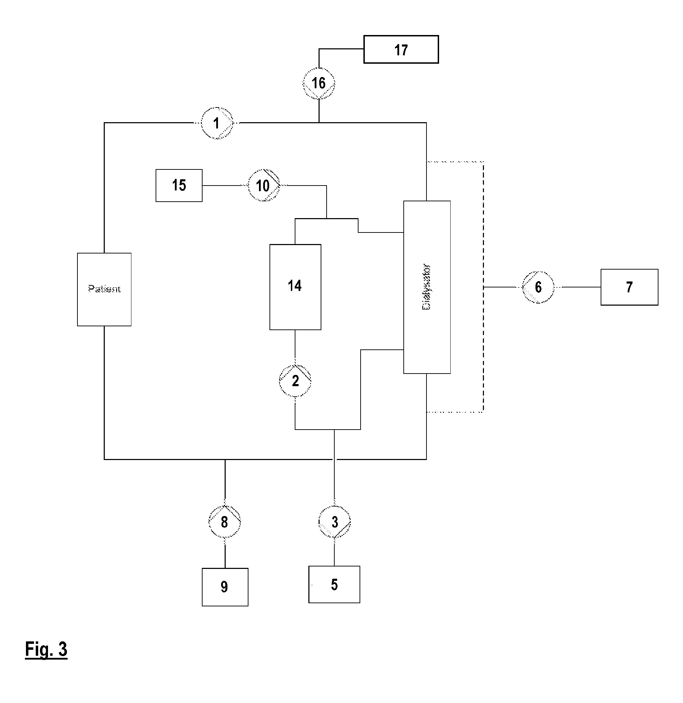

Fig. 3 zeigt schematisch eine weitere Ausführungsform eines erfindungsgemäßen Dialysesystems, die für einen Einsatz in der Heimdialyse oder für einen Einsatz als tragbares Dialysesystem geeignet ist. Auf Grund ihrer geringen Baugröße, ihres geringen Gewichts und der geringen Stromaufnahme eignen sich Piezopumpen besonders für die Konstruktion tragbarer Dialysesysteme, da dadurch eine kompakte und leichte Bauweise der Vorrichtung ermöglicht wird und eine Stromversorgung über Akkumulatoren bzw. Batterien möglich ist, ohne dass dadurch das Gewicht des Gesamtsystems zu groß wird. Eine Piezopumpe (**1**) ist dafür vorgesehen, das Blut des Patienten durch den extrakorporalen Kreislauf zu pumpen. Piezopumpe (**2**) ist dafür vorgesehen, Dialysat durch den Dialysatkreislauf, der in dieser Ausführungsform neben dem Dialysator auch Mittel zur Dialysatregeneration (**14**) aufweist, zu pumpen. Piezopumpe (**3**) ist dafür vorgesehen, aus dem Dialysatkreislauf ausgeschleustes verbrauchtes Dialysat in einen Behälter (**5**) für verbrauchtes Dialysat zu pumpen. Dem Dialysat wird in dieser Ausführungsform nach der Regeneration aus einer Quelle (**15**), z.B. einem Vorratsbehälter, Elektrolytlösung über Piezopumpe (**10**) beigemischt, um den Elektrolytgehalt des Dialysats zu regulieren. Eine weitere Piezopumpe (**6**) ist dafür eingerichtet, bei Bedarf Substitutionsflüssigkeit aus einer Quelle (**7**), z.B. einem Vorratsbehälter, dem extrakorporalen Blutkreislauf zuzuführen, z.B. wenn das Dialysesystem im Hämodiafiltrationsmodus (HDF) oder im Hämofiltrationsmodus (HF) betrieben wird, wahlweise vor dem Dialysator ("pre-dilution mode") oder hinter dem Dialysator ("post-dilution mode"). Eine weitere Piezopumpe (**8**) ist dafür vorgesehen, Infusionslösung (beispielsweise Medikamentenlösungen) aus einer Quelle (**9**) dem extrakorporalen Blutkreislauf zuzuführen. Eine weitere Piezopumpe (**16**) ist dafür vorgesehen, dem Blut des Patienten im extrakorporalen Blutkreislauf vor dem Eintritt in den Dialysator gerinnungshemmende Mittel wie Heparin aus einer entsprechenden Quelle (**17**) zuzuführen.

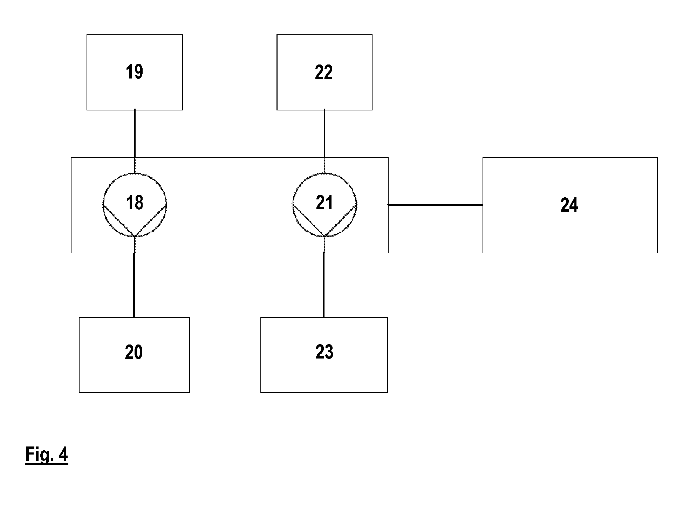

Fig. 4 zeigt schematisch ein Zusatzmodul für ein Dialysesystem, das für die Steuerung der Blutgerinnung während der Dialyse eingesetzt werden kann. Eine Piezopumpe (**18**) ist dafür vorgesehen, Citratlösung aus einer Quelle (**19**), z.B. einem Vorratsbehälter, in einen arteriellen Blutschlauch (**20**) zu pumpen, um die Gerinnungsneigung des Blutes des Patienten vor dem Eintritt in den Dialysator herabzusetzen. Eine weitere Piezopumpe (**21**) ist dafür vorgesehen, eine Calciumionen enthaltende Lösung aus einer Quelle (**22**), z.B. einem Vorratsbehälter, in einen venösen Blutschlauch (**23**) zu pumpen, um die Gerinnungsneigung des Blutes des Patienten nach Verlassen des Dialysators wieder zu erhöhen. Mittel (**24**) zur Steuerung der Piezopumpen (**18,21** sind zur Steuerung der Pumpenfunktion vorgesehen. Es kann sich dabei z.B. um eine Schnittstelle handeln, die das Zusatzmodul mit dem Dialysemonitor verbindet. Dies ermöglicht die Synchronisierung der Funktion der Piezopumpen (**18,21**) untereinander wie auch mit den Flussraten im extrakorporalen Blutkreislauf. Die Verbindung des Zusatzmoduls mit dem Dialysemonitor kann beispielsweise über Kabel oder drahtlos erfolgen, z.B. über W-LAN.

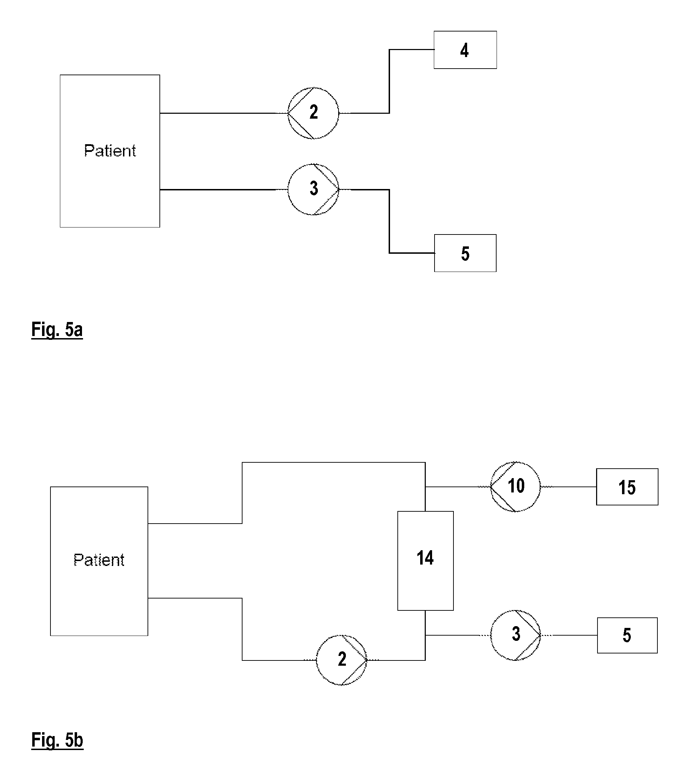

Fig. 5a und 5b zeigen schematisch weitere Ausführungsformen eines erfindungsgemäßen Dialysesystems, die für einen Einsatz in der Peritonealdialyse geeignet sind. Fig. 5a zeigt eine Ausführungsform, in der Piezopumpe (**2**) dafür vorgesehen ist, Dialysat aus einer Dialysatquelle (**4**), z.B. einem Beutel, in den Bauchraum des Patienten zu pumpen und Piezopumpe (**3**) dafür vorgesehen ist, das den Bauchraum des Patienten verlassende Dialysat in einen Behälter (**5**) für verbrauchtes Dialysat zu pumpen. Fig. 5b zeigt eine Ausführungsform, in der Piezopumpe (**2**) dafür vorgesehen ist, Dialysat durch den Dialysatkreislauf, der in dieser Ausführungsform Mittel zur Dialysatregeneration (**14**) aufweist, zu pumpen. Piezopumpe (**3**) ist dafür vorgesehen, aus dem Dialysatkreislauf ausgeschleustes verbrauchtes Dialysat in einen Behälter (**5**) für verbrauchtes Dialysat zu pumpen. Dem Dialysat wird in dieser Ausführungsform nach der Regeneration aus einer Quelle (**15**), z.B. einem Vorratsbehälter, Elektrolyte und Glukose enthaltende Lösung über Piezopumpe (**10**) beigemischt, um den Elektrolyt- und Glukosegehalt des Dialysats zu regulieren.

## Patentansprüche

1. Dialysesystem umfassend mindestens eine peristaltische Schlauchpumpe linearer Bauart mit piezoelektrischen Aktuatoren.

2. Dialysesystem gemäß Anspruch 1, umfassend mindestens eine peristaltische Schlauchpumpe linearer Bauart mit piezoelektrischen Aktuatoren (**1**), die eingerichtet ist zum Pumpen von Blut.

3. Dialysesystem gemäß Anspruch 1 oder 2, umfassend mindestens eine peristaltische Schlauchpumpe linearer Bauart mit piezoelektrischen Aktuatoren (**2,3**), die eingerichtet ist zum Pumpen von Dialysat.

4. Dialysesystem gemäß einem der Ansprüche 1 bis 3, umfassend mindestens eine peristaltische Schlauchpumpe linearer Bauart mit piezoelektrischen Aktuatoren (**6**), die eingerichtet ist zum Pumpen von Substitutionsflüssigkeit.

5. Dialysesystem gemäß einem der Ansprüche 1 bis 4, umfassend mindestens eine peristaltische Schlauchpumpe linearer Bauart mit piezoelektrischen Aktuatoren (**8**), die eingerichtet ist zum Pumpen von Infusionsflüssigkeit.

6. Dialysesystem gemäß einem der Ansprüche 1 bis 5, umfassend mindestens eine peristaltische Schlauchpumpe linearer Bauart mit piezoelektrischen Aktuatoren (**10,11**), die eingerichtet ist zum Pumpen von Dialysatkonzentrat.

7. Dialysesystem umfassend einen Dialysator, der an einen extrakorporalen Blutkreislauf und einen Dialysatkreislauf angeschlossen ist, wobei im extrakorporalen Blutkreislauf eine peristaltische Schlauchpumpe linearer Bauart mit piezoelektrischen Aktuatoren (**1**) zum Pumpen von Blut angeordnet ist; der extrakorporale Blutkreislauf optional über eine peristaltische Schlauchpumpe linearer Bauart mit piezoelektrischen Aktuatoren (**6**) an eine Quelle für Substitutionsflüssigkeit (**7**) angeschlossen ist; der extrakorporale Blutkreislauf optional über eine peristaltische Schlauchpumpe linearer Bauart mit piezoelektrischen Aktuatoren (**8**) an eine Quelle für Infusionsflüssigkeit (**9**) angeschlossen ist; der extrakorporale Blutkreislauf optional über eine peristaltische Schlauchpumpe linearer Bauart mit piezoelektrischen Aktuatoren (**16**) an eine Quelle für Koagulationshemmer (**17**) angeschlossen ist; wobei im Dialysatkreislauf mindestens eine peristaltische Schlauchpumpe linearer Bauart mit piezoelektrischen Aktuatoren (**2,3**) zum Pumpen von Dialysat angeordnet ist; der Dialysatkreislauf optional über eine peristaltische Schlauchpumpe linearer Bauart mit piezoelektrischen Aktuatoren (**10,11**) an eine Quelle für Dialysekonzentrat (**12,13**) oder Elektrolytlösung (**15**) angeschlossen ist; und im Dialysatkreislauf optional Mittel zur Dialysatregeneration (**14**) angeordnet sind.

8. Vorrichtung für die Peritonealdialyse, umfassend einen Dialysatkreislauf, in dem mindestens eine peristaltische Schlauchpumpe linearer Bauart mit piezoelektrischen Aktuatoren (**2,3**) zum Pumpen von Dialysat angeordnet ist; der Dialysatkreislauf optional über eine peristaltische Schlauchpumpe linearer Bauart mit piezoelektrischen Aktuatoren (**10**) an eine Quelle für Elektrolyt- und Glucoselösung (**15**) angeschlossen ist; und im Dialysatkreislauf optional Mittel zur Dialysatregeneration (**14**) angeordnet sind.

9. Vorrichtung zur Steuerung der Blutgerinnung, umfassend eine Quelle für Citratlösung (**19**), die verbunden ist mit einer peristaltischen Schlauchpumpe linearer Bauart mit piezoelektrischen Aktuatoren (**18**), die wiederum verbunden ist mit einem arteriellen Blutschlauch (**20**); eine Quelle für Calciumionen enthaltende Lösung (**22**), die verbunden ist mit einer peristaltischen Schlauchpumpe linearer Bauart mit piezoelektrischen Aktuatoren (**21**), die wiederum verbunden ist mit einem venösen Blutschlauch (**23**); sowie Mittel (**24**) zur Steuerung der Schlauchpumpen (**18,21**)

10. Verwendung einer peristaltischen Schlauchpumpe linearer Bauart mit piezoelektrischen Aktuatoren in Dialysesystemen.
